# EUROPÄISCHE PATENTSCHRIFT

(11) **EP 2 934 437 B1**
(45) Veröffentlichungstag und Bekanntmachung des Hinweises auf die Patenterteilung: **15.08.2018**
(21) Anmeldenummer: 13802061.5
(22) Anmeldetag: 10.12.2013
(51) Int. Cl.: A61K 8/26, A61K 8/27, A61K 8/33, A61Q 15/00

(54) **VERWENDUNG VON ALUMINIUMSALZEN GEGEN STRESS-SCHWITZEN**
USE OF ALUMINIUM SALTS AGAINST STRESS PERSPIRATION
UTILISATION DE SELS D'ALUMINIUM POUR LUTTER CONTRE LA TRANSPIRATION DUE AU STRESS

(30) Priorität: 18.12.2012 DE 102012223553
(43) Veröffentlichungstag der Anmeldung: 28.10.2015
(73) Patentinhaber: Beiersdorf AG, 20253 Hamburg (DE)
(72) Erfinder: BIEL, Stefan, 21077 Hamburg (DE); URBAN, Michael, 22523 Hamburg (DE); SCHMIDT-ROSE, Thomas, 22605 Hamburg (DE); WINDISCH, Björna, 22607 Hamburg (DE); BÜRGER, Anette, 22529 Hamburg (DE)
(74) Vertreter: Hartmann, Jost
(86) Internationale Anmeldenummer: PCT/EP2013/076023
(87) Internationale Veröffentlichungsnummer: WO 2014/095475

(56) Entgegenhaltungen:
- DE-T3-602005 000 901
- GB-A- 1 533 048
- US-A1- 2007 248 553
- US-B1- 6 403 067
- WENZEL ET AL: "Nonneoplastic disorders of the eccrine glands", JOURNAL OF THE AMERICAN ACADEMY OF DERMATOLOGY, C.V. MOSBY, ST. LOUIS, MO, US, Bd. 38, Nr. 1, 1. Januar 1998 (1998-01-01) , Seiten 1-20, XP005686606, ISSN: 0190-9622
- ANTRANIK BENOHANIAN: "Antiperspirants and deodorants", CLINICS IN DERMATOLOGY, Bd. 19, Nr. 4, 1. Juli 2001 (2001-07-01), Seiten 398-405, XP055123589, ISSN: 0738-081X, DOI: 10.1016/S0738-081X(01)00192-4
- DATABASE EMBASE [Online] ELSEVIER SCIENCE PUBLISHERS, AMSTERDAM, NL; 2005, HRASTIC I ET AL: "Antiperspirants and deodorants", XP002720939, Database accession no. EMB-2005497057 & HRASTIC I ET AL: "Antiperspirants and deodorants", FARMACEUTSKI GLASNIK 2005 HR, Bd. 61, Nr. 10, 2005, Seiten 557-570, ISSN: 0014-8202
- DATABASE GNPD [Online] MINTEL; 1. Oktober 2008 (2008-10-01), "Deodorant Roll-On", XP002720901, Database accession no. 989929
- DATABASE GNPD [Online] MINTEL; 1. November 2012 (2012-11-01), "Antiperspirant Deodorant Roll-On", XP002720902, Database accession no. 1934671
- KANLAYAVATTANAKUL AND N LOURITH M: "Body malodours and their topical treatment agents", INTERNATIONAL JOURNAL OF COSMETIC SCIENCE, KLUWER ACADEMIC PUBLISHERS, DORDRECHT, NL, 1. Januar 2011 (2011-01-01), Seiten 1-14, XP007917929, ISSN: 0142-5463, DOI: 10.1111/J.1468-2494.2011.00649.X [gefunden am 2011-03-15]
- KIRSCHBAUM ET AL: NEUROPSYCHOBIOLOGY, 31 December 1993 (1993-12-31),
- HELLHAMMER ET AL: INT. J. COSM. SCI, 31 December 2011 (2011-12-31),

## Beschreibung

Die Erfindung ist die Verwendung von Aluminiumsalzen in Kombination mit ein oder mehreren Adjuvantien in kosmetischen oder dermatologischen Zubereitungen zur Verminderung oder Verhinderung der apoekkrinen Schweißbildung.

Als Schweiß wird ein von der Haut des Menschen über so genannte Schweißdrüsen abgesondertes wässriges Sekret bezeichnet. Es gibt drei Arten von Schweißdrüsen in der Haut, nämlich apokrine, ekkrine und apoekkrine Schweißdrüsen (Int J Cosmet Sci. 2007 Jun; 29(3):169-79).

Die ekkrinen Schweißdrüsen sind beim Menschen praktisch über den ganzen Körper verteilt und können beträchtliche Mengen eines klaren, geruchlosen Sekretes produzieren, das zu über 99% aus Wasser besteht. Im Gegensatz dazu kommen die apokrinen Schweißdrüsen nur in den behaarten Körperarealen der Achsel- und Genitalregion sowie an den Brustwarzen vor. Sie produzieren geringe Mengen eines milchigen Sekretes, das Proteine und Lipide enthält und chemisch neutral ist.

Das Schwitzen, auch als Transpiration bezeichnet, ist ein effektiver Mechanismus, um überschüssige Wärme abzugeben und damit die Körpertemperatur zu regulieren. Hierzu dient vor allem das volumenreiche wässrige Sekret der ekkrinen Drüsen, die beim Erwachsenen bis zu 2-4 Liter pro Stunde bzw. 10-14 Liter am Tag produzieren können.

Dem Schweiß - insbesondere dem Sekret der apokrinen Schweißdrüsen - wird darüber hinaus eine Signalwirkung über den Geruchssinn zugesprochen. Beim Menschen spielt der apokrine Schweiß insbesondere im Zusammenhang mit dem emotionalen oder stressbedingten Schwitzen eine Rolle.

Kosmetische Antitranspirantien oder Desodorantien/Deodorantien dienen dazu, Körpergeruch zu beseitigen bzw. deren Entstehung zu vermindern. Körpergeruch entsteht, wenn der an sich geruchlose frische Schweiß durch Mikroorganismen wie z.B. Staphylokokken und Corynebakterien zersetzt wird.

Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

AT- Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen gram-positive und gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Gängige Wirkmechanismen hierzu sind antibakterielle Effekte, wie sie z.B. auch das nicht-kolloidale Silber zeigt, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Reaktionen zu wohlriechenden Stoffen umgesetzt werden.

Schweißgeruch besteht zu einem Großteil aus verzweigt-kettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren. Häufig wirken die dabei zum Einsatz kommenden Substanzen jedoch unselektiv auch gegen nützliche Hautkeime und können bei empfindlichen Personen zu Hautirritationen führen.

Die Wirkung von Antitranspirantien auf Basis von Al-Salzen gegen thermisches Schwitzen unter normalen physiologischen Bedingungen ist sehr gut untersucht.

Es wird angenommen, dass die Schweißreduktion bei Aluminium-haltigen Antitranspirantwirkstoffen u.a. durch eine "Verstopfung der Schweißdrüse" erzielt wird. Sie wirken durch Verstopfung von Schweißdrüsenausfuhrgängen, indem sie vor Ort zusammen mit hauteigenen Proteinen ausfallen und so zu sogenannten Plugs führen.

Ob diese Verstopfung durch Denaturierung des Keratins oder durch Verklumpung von Korneozyten im Schweißdrüsengang verursacht wird (Shelley WB and Hurley HJ, Acta. Derm. Venereol. (1975) 55: 241-60), oder durch die Entstehung eines ACH/AZG-Gels (Reller HH and Luedders WL, in: Advances in Modern Toxicology, Dermatoxicology and Pharmocology, F.N. Marzulli and H.I. Maibach, Eds. Hemisphere Publishing Company, Washington and London (1977) Vol. 4: 1-5), das durch Neutralisation im Schweißdrüsenausführgang gebildet wird, ist nach wie vor offen.

Die so erzielte und bekannte Verstopfung ist allerdings nur kurzfristig wirksam. Starkes Schwitzen oder die Reinigung der Achsel im Rahmen der normalen Körperreinigungsroutine heben die Verstopfung wieder auf und somit auch den Antitranspiranteffekt. Die daraus resultierende Notwendigkeit, Antitranspirant (AT)-Produkte mindestens einmal täglich aufzutragen führt aber ggf. zu Hautreizungen, speziell nach der Rasur oder in oder an vorgeschädigten Hautarealen.

In WO 2007071474 A1 wird außerdem beschrieben, dass die axilläre Darreichung von Aluminium-haltigen Antitranspirantien, vorzugsweise ACH, in Deo/AT-Formulierungen durch Zugabe von geringen Mengen organischer Lösungsmittel zu einer stark erhöhten Keratinisierungsrate der Zellen des Schweißdrüsenausführgangs führt. In der Folge entstehen vermehrt verhornte Korneozyten, die im Zuge der natürlichen Desquamation in den Dukt hinein abgegeben werden und so den Dukt von innen heraus verstopfen und den Schweißfluss verhindern. Dieser zusätzliche Verstopfungsmechanismus ist unempfindlich gegen vermehrtes Schwitzen und/oder Reinigung und stellt somit einen zusätzlichen Langzeit-Antitranspirant (AT)-Effekt dar.

Neben dieser vornehmlich thermisch bedingten Schweißbildung und den entsprechenden Gegenmaßnahmen stand das sogenannte Stress-Schwitzen bislang wenig im Vordergrund.

In der Literatur (Effective prevention of stress-induced sweating and axillary malodour formation in teenagers (Int J Cosmet Sci. 2011 Feb;33(1):90-7) wurde sich mit dem Problem des emotionalem Schwitzens beschäftigt. Emotionales Schwitzen und der damit einhergehende verstärkte schlechte Geruch stellt eine relevante Herausforderung für die heutigen Antitranspirants (AT) und Deodorants dar. Es wurde das Stress-induzierte Schwitzen bei Jugendlichen untersucht, indem klassische Stresssituationen nachgestellt wurden, wie Prüfungen in der Schule oder Vorstellungsgespräche.

EP 1541122 A1 beschreibt Zink PCA (Pidolate) in AT-Formulierungen und den Synergismus hinsichtlich der Schweißgeruchsreduktion bei Kombination von ACH mit Zinkpidolat im Verhältnis Zinkpidolat/ACH von 1/20 bis 5/1. Informationen zum Stress-Schwitzen bzw. apoekkriner Schweißbildung werden nicht geliefert.

EP 768080 B1 beschreibt desodorisierende Zusammensetzungen in Form von W/Si-Emulsionen, die ein wasserlösliches Zinksalz als Geruchsabsorber enthalten.

EP 1833414 A2 beschreibt die Kombination von mindestens zwei wasserlöslichen Zinksalzen zu Konzentrationen von in Summe mindestens 0,1 % sowie die Kombination mit verschiedenen antimikrobiell wirksamen Stoffen.

US 5662937 beschreibt Deodorant-Zubereitungen enthaltend modifizierte Stärken als Substitut für Talkum unter Zusatz von Zinkoxid und Citronensäure.

WO 2007076125 A2 beschreibt antimikrobiell wirksame Zubereitungen, die eine ZinkVerbindung enthalten.

DE 102005032239 A1 offenbart AT-Puder mit einem Applikationspad. In einigen Beispielen wird Zinkstearat oder Aluminumstearat eingesetzt und dienen dort als Trennmittel in der Pudergrundlage. Informationen zum Stress-Schwitzen bzw. apoekkriner Schweißbildung werden nicht geliefert.

DE 102006004957 A1 beschreibt Deo/AT-Stifte auf Emulsionsbasis mit keinerlei Bezug zu Stress-Schwitzen bzw. apoekkriner Schweißbildung.

DE 102010055816 A1 beschreibt Deo/AT-Sprays mit FKW-Treibmitteln. Es werden allgemein AT-Wirker und deren Kombinationen sowie Zink-Salze als Deo-Wirker beschrieben sowie ein Auflistung von Parfümkomponenten.

DE 699 28 378 T2 beschreibt Zubereitungen mit duftverstärkenden Komponenten, die u.a. auch aldehydisch sein können und mit AT-Mitteln kombiniert werden können. Der beschriebene Effekt führt zu einer Duftsteigerung und behandelt weder die Problematik des Stress-Schwitzens noch das Thema der Verminderung der apoekkrinen Schweißbildung.

In der US 7 763 238 B2 wird die sog. "Neo Fresh"-Technologie beschrieben. Hierbei handelt es sich um Desodorierung, d.h. Verbesserung des Schlechtgeruches, und nicht um Antitranspiratwirkung. Genauer gesagt wird dort die Entstehung von Schlechtgeruch nicht einmal verhindert oder reduziert, sondern lediglich dessen Wahrnehmbarkeit verringert

Im Markt sind übliche Deodorantien erhältlich, die mit einer Stressresistenz werben, z.B. Fa Deo Roll-on Active Pearls, Vichy Stress Resist 72 h. Letzteres Produkt soll eine 72 Stunden Schweißregulierung sogar in Stress-Situationen bieten. Die Anwender selber zeigen dabei zu 74% eine Stresssituation durch Selbsteinschätzung an.

Gegen die übermäßige Geruchsentwicklung beim Stress-Schwitzen werden ansonsten parfumhaltige Zubereitungen als Lösung angeboten (z.B. secret® clinical strength). Nachteilig an den bislang bekannten Anwendungen und Produkten ist, dass ihre Produktleistung nicht verifizierbar hinsichtlich des emotional begründeten Schwitzens sind. Aufgrund der Datenlage zum Vorhandensein der verschiedenen Schweißdrüsentypen (ekkrin, apokrin, apoekkrin) in der humanen Achsel sowie des bekannten Effekts, dass Stressschweiß eine deutlich intensivere olfaktorische Signatur hinterlässt als "normaler" Schweiß, ist davon auszugehen, dass unter Stressbedingungen die Stimulation der geruchsabsondernden apo- und apoekkrinen Schweißdrüsen stärker ist als unter Normalbedingungen.

Aufgrund dieser Problemstellung ist es wünschenswert Produkte zur Verfügung zu stellen, die diesem veränderten Wirkspektrum Rechnung tragen.

Um sicherzustellen, dass es sich bei untersuchten Schweißreduktionen um realen Stressschweiß handelt, der auf eine entsprechende Erhöhung der Stress-abhängigen Signalstoffe im Körper zurückzuführen ist, reicht deshalb eine einfache Probandenbefragung nicht aus. Vielmehr ist es notwendig, hierfür ein wissenschaftlich validiertes Verfahren heranzuziehen, das die Absonderung von Stressschweiß unter standardisierten Bedingungen und mit biochemischer Kontrolle der Stress-relevanten Botenstoffe sicher stellt. Ansonsten besteht die Gefahr, dass die Probanden durch die Art der Befragung oder die Erwartungshaltung der Fragenden dazu verleitet werden, normales thermisches Schwitzen als Stressschwitzens zu benennen und damit die Studienergebnisse zu verfälschen.

Die Verwendung eines wissenschaftlich anerkannten Verfahrens zur reproduzierbaren Erzeugung von Stress und Stressschweiß ermöglicht dagegen erstmals die gezielte Entwicklung von Produkten bzw. Antitranspirantien, die über das thermische Schwitzen der ekkrinen Schweißdrüsen hinaus auch die stress-bedingt erhöhte Schweißsekretion der apokrinen Schweißdrüse und vor allem der apoekkrinen Schweißdrüse reduzieren können, deren zu erwartende Schweißrate die der apokrinen Schweißdrüse deutlich übersteigen wird. Dieses neue Target für Antitranspirantien kann damit durch dieses Untersuchungsverfahren erstmals zur Untersuchung erfindungsgemäßer Zubereitungen gezielt adressiert werden.

Wünschenswert ist es daher gezielte Mechanismen des Stress-Schwitzens zu erkennen und damit Lösungen gegen Stress-Schwitzen und den damit einhergehenden Geruch anzubieten.

Die Erfindung ist die Verwendung von Aluminiumsalzen in Kombination mit ein oder mehreren Adjuvantien in kosmetischen oder dermatologischen laut Anspruch 1. Die Verwendung von Aluminiumsalzen in Kombination mit ein oder mehreren Adjuvantien laut Anspruch 1 in kosmetischen oder dermatologischen Zubereitungen ermöglicht die Verminderung oder Verhinderung des Stress-Schwitzens entsprechend dem TSST-Design.

Die Kombination aus Aluminiumsalzen in Kombination mit den geeigneten Adjuvantien in kosmetischen oder dermatologischen Zubereitungen bietet eine wirksamere Anwendung gegen apoekkrines bzw. Stress-Schwitzen.

Die Kombination aus Aluminiumsalzen und geeigneten Adjuvantien wird in kosmetischen oder dermatologischen Zubereitungen, insbesondere zur topischen Applikation, bereit gestellt.

Mit der erfindungsgemäßen Verwendung der Kombination aus Aluminiumsalzen und Adjuvantien wird erstmalig gezielt den besonderen Bedürfnisse des apoekkrinen Schwitzens Rechnung getragen. Diese besonderen Bedürfnisse begründen sich in einer
- abweichenden Schweiß-Zusammensetzung des apoekkrinen Schweißes (lipophilerer Charakter aufgrund der Vermischung von ekkrinem, wässrigen Schweiß mit apokrinem lipophilen Schweiß beim apoekkrinen Sekretionsprozess)
- anderen Drüsengeometrie der apoekkrinen Drüse im Vergleich zur ekkrinen Drüse (größeres Lumen der Schweißdrüse, zum Teil Öffnung des Ausführganges in den Haarschaft)
Das oder die Aluminium-Salze sind
- Aluminiumchlorhydrat [Al₂(OH)₅Cl] x H₂O
   ∘ Standard Al-Komplexe: Locron P (Clariant), Micro-Dry (Reheis), ACH-331 (Summit), Aloxicoll PF 40 (Giulini).
   ∘ Aktivierte Al-Komplexe: Reach 501 (Reheis), AACH-324 (Summit), AACH-7171 (Summit), Aloxicoll P (Giulini), Aloxicoll SD100

Die Aluminiumsalze aus den zuvor geschilderten Gruppen werden in den erfindungsgemäßen Formulierungen bevorzugt in einer Menge von 1 bis 30 Gew.%, bezogen auf die Gesamtmasse der Zubereitung, d.h. inklusive der ggf. vorhandenen Treibgase, eingesetzt.

Bei Einsatz von z.B. ca. 33,3 Gew.% ACH (Aluminium Chlorohydrate, 50 % aq.) in der Wirkstofflösung für ein Aerosol-Spray (ohne Treibgas) und einem Abfüllverhältnis von etwa 30 : 70 (Wirkstofflösung zu Treibgas) wird ein Anteil von etwa 5 Gew. % ACH im Endprodukt vorhanden sein.

Als Wirkstofflösung wird die Summe aller Bestandteile ohne das Treibgas bezeichnet. Der klassische Wirkmechanismus von antitranspirantwirksamen Aluminiumsalzen, wie Aluminiumchlorohydrat (ACH), beruht auf einer Verstopfung des Schweißdrüsenausführgangs der ekkrinen Schweißdrüse, u.a. durch Verkleben von abgeschilferten Korneozyten des verhornten Ausführganges. Da der apoekkrine Schweißdrüsenausführgang zum Teil auch in den Haarschaft mündet und damit nicht direkt an der Hautoberfläche endet, ist dieser Wirkmechanismus für diese Schweißdrüsenpopulation weniger wahrscheinlich. Aus diesem Grund ist es überraschend, dass die Kombination eines klassisches AT-Wirkers (Aluminium-Salze) mit Adjuvantien, die die AT-Wirker-vermittelte Verklebung von losen Korneozyten mit Hautfetten und anderen Körper-eigenen Substanzen, z.B. über Komplexierung, ermöglichen, eine deutliche Reduzierung auch des stressbedingten, d.h. apoekkrinen Schwitzens ermöglichen. Diese Haftvermittlung zwischen Schweiß, Haut, Hautfett und AT-Mittel beruht auf hydrophilhydrophoben Wechselwirkungen zwischen den Al-Salz-Komplexen des AT-Mittels auf der einen und den hydrophoben, die Korneozyten-Oberflächen bedeckenden Hautfetten auf der anderen Seite; diese Wechselwirkungen können durch die Gegenwart freier, mehrwertig positiv geladener Metallionen verstärkt werden, ebenso wie durch die Zugabe von Komplexbildnern, die an Al-Ionen oder andere, mehrwertig positiv geladene Metallionen binden und so den Plug vergrößern. Dieser Mechanismus kann darüber hinaus durch Zugabe anderer, in pH-Wert und Olationsgrad vom klassischen ACH sich unterscheidender Al-basierter AT-Mittel weiter verstärkt werden.

Bevorzugte Adjuvantien können weiterhin ausgewählt werden aus der Gruppe der Zink-Salze, insbesondere derer von Carbonsäuren.

Besonders bevorzugt sind Zinksalze der kurzkettigen Mono-, Di- und Tri-Carbonsäuren, wie u.a. Essigsäure, Milchsäure und der Gruppe der Fruchtsäuren, insebsondere Oxalsäure, Bernsteinsäure, Fumarsäure, Maleinsäure und Citronensäure.

Ganz besonders bevorzugt ist Zinkcitrat.

Der Anteil an ein oder mehreren Zinksalzen wird vorteilhaft im Bereich von 0,005 - 5 Gew.%, bevorzugt 0,01 - 0,5 Gew.%, bezogen auf die Gesamtmasse der Zubereitung, gewählt.

Vorteilhaft wird das Gewichtsverhältnis zwischen antitranspirantwirksamer Aluminiumverbindungen und Zinksalzen im Bereich von 4000 zu 1 bis 2 zu 1 gewählt.

So zeigte sich beispielsweise 0,01 Gew.% Zinkcitrat und 35 Gew.% ACH oder 0,1 Gew.% Zinkcitrat und 35 Gew.% ACH und 5 Gew.% ASCH als besonders vorteilhaft gegen Stress-Schwitzen ebenso wie eine Kombination von 0,3 Gew.% ACH und 0,1 Gew.% Zinkcitrat vorteilhaft. Als Aluminumsalz wird Aluminiumchlorohydrat (ACH) und als Adjuvans c. Aluminium Sesquichlorohydrate (ASCH) gewählt.

Die als Aluminiumsalze zu wählenden Adjuvantien c. unterscheiden sich von dem als erfindungsgemäß zwingend vorhandenem Aluminiumsalz.

Durch die Kombination zweier verschiedener Aluminiumsalze wird ein stabilisierendes Puffersystem generiert, das den pH-Wert im für die Wirksamkeit des Stress-Schwitzens optimalen Bereich hält.

Insbesondere hat sich die Kombination aus ACH und ASCH als besonders geeignet erwiesen, da sich durch diese Kombination ohne signifikante Veränderung des pH-Werts das Al/Cl-Verhältnis innerhalb der Formulierung beeinflussen lässt. Dadurch kann eine optimale Mischung verschiedener anionischer Spezies zur Verfügung gestellt werden, um den gewünschten Effekt zu erzeugen. Aluminium Sesquichlorhydrat wird bevorzugt in einem Gewichtsverhältnis zum vorhandenen Aluminiumsalz im Bereich von 1% - 25 %, insbesondere 5 - 15 %, gewählt. So ist bei einem vorteilhaften Gewichtsanteil an Aluminiumchlorohydrat von 10 Gew.%, bezogen auf die Gesamtmasse der Zubereitung, das zweite Aluminiumsalz als Adjuvans ASCH, im Verhältnis dazu von 10 % zu wählen, also 10% von 10 Gew.% sind 1 Gew.% ASCH. Erfindungsgemäße Kombination aus Aluminiumsalz und Adjuvantien zielt erstmalig auf die besonderen Bedürfnisse des apoekkrinen Schwitzens.

Als erfindungsgemäß vorteilhaft, können AT-Wirker auch mit mehreren Adjuvantien ausgewählt aus den Gruppen a. bis c. miteinander kombiniert werden. Also beispielsweise als Aluminiumsalz und AT-Wirker Aluminiumchlorohydrat (ACH) und als Adjuvans aus der Gruppe c zum Beispiel Aluminium Sesquichlorohydrate in Kombination mit einem weiteren Adjuvans aus der Gruppe der Zinksalze (b). Ganz besonders bevorzugt ist die Kombination von Aluminiumchlorohydrat (ACH) mit Aluminium Sesquichlorohydrate und Zinkcitrat.

Gleichermaßen können alle drei Adjuvans-Gruppen mit dem AT-Wirker kombiniert werden.

Der Trier Social Stress Test (TSST) gilt als das zuverlässigste und standardisierte Stress-Protokoll.

Die TSST ist ein Verfahren, das an der Universität Trier entwickelt wurde und mittlerweile als Standard für die Induktion des moderaten psychosozialen Stresses unter Laborbedingungen gilt. Das zentrale Element des TSST ist eine Situation, die durch Neuheit, Unsicherheit, Unkontrollierbarkeit und einer sozial-evaluative Bedrohung gekennzeichnet ist (Kirschbaum, C., Pirke, K., & Hellhammer, D.H. The "Trier social stress test" - a tool for investigating psychobiological stress response in a laboratory setting. Neuropsychobiology. 28, 76-81 (1993)).

Nachfolgende Beispiele erläutern die erfindungsgemäßen Zubereitungen, die zur Verminderung oder Verhinderung der apoekkrinen Schweißbildung und des Stress-Schwitzens entsprechend dem TSST-Design verwendet werden können.

Die Zahlenangaben stellen Gewichtsanteile, bezogen auf die Gesamtmasse der Zubereitung, dar.

### Beispiele

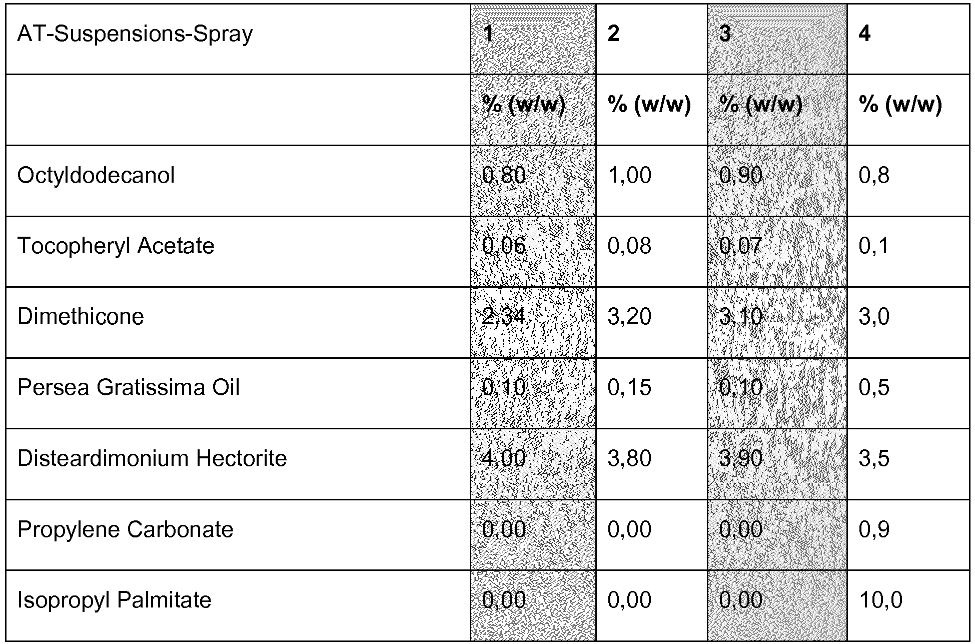

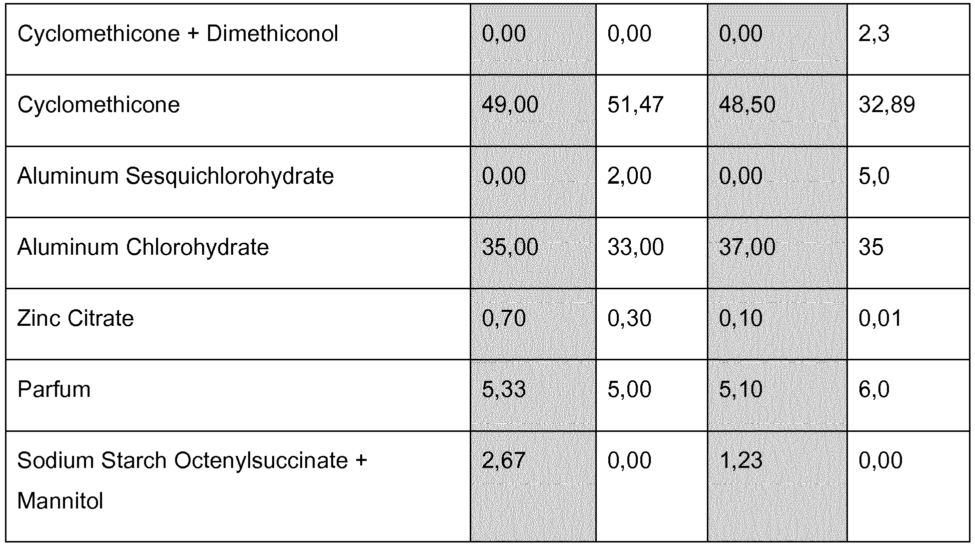

Mit Treibgas vorzugsweise Butane/Isobutane/Propane und Abfüllverhältnis 5:95 bis 30:70, vorzugsweise 10:90 bis 20:80, vorzugsweise 15:85.

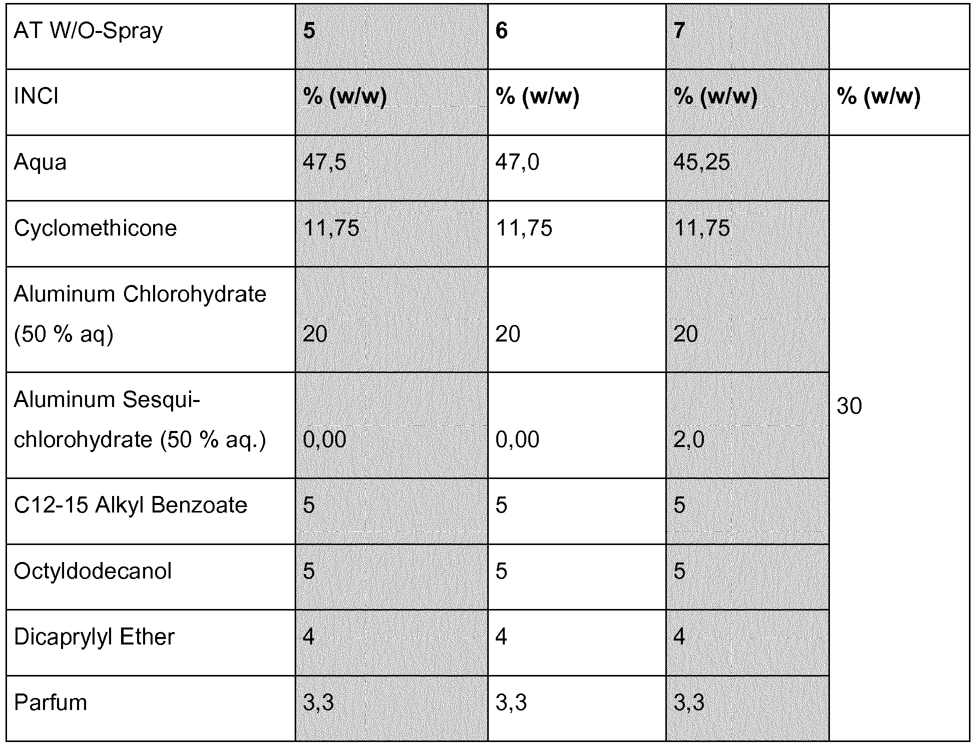

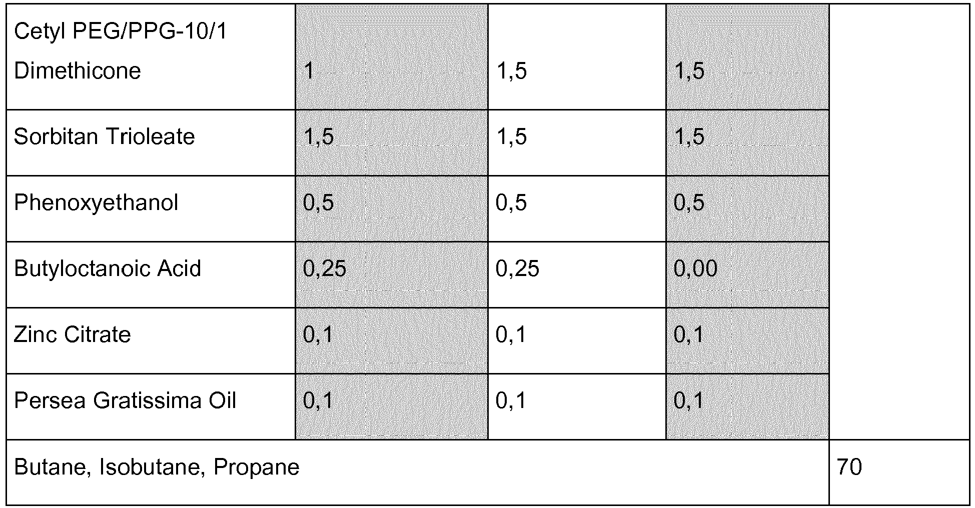

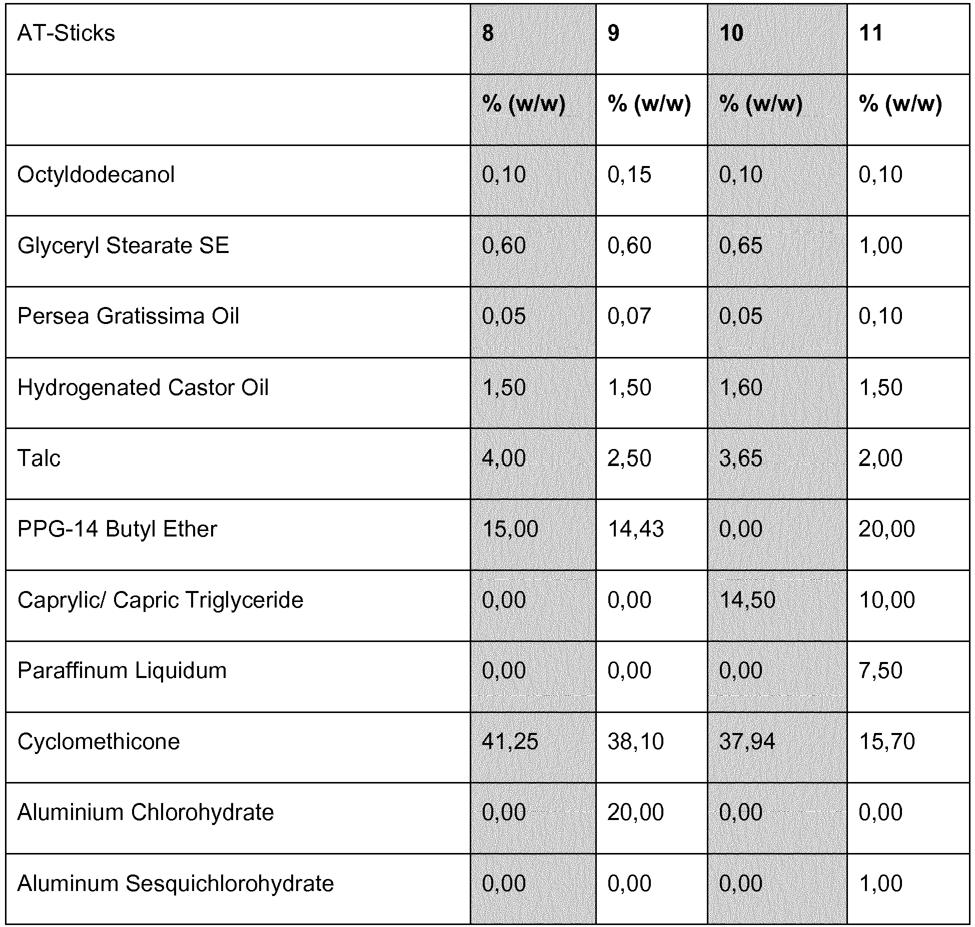

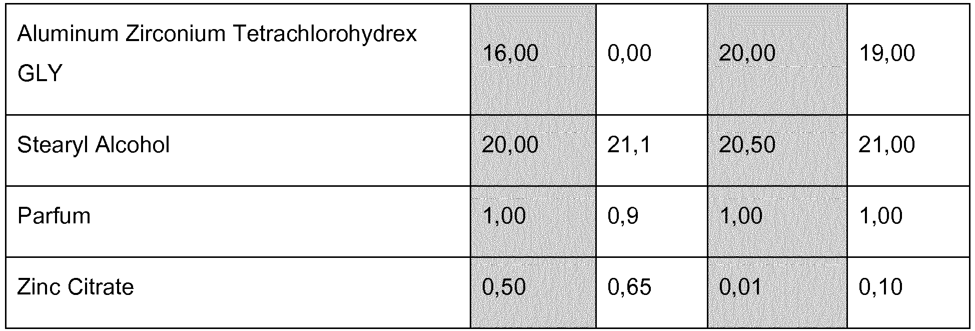

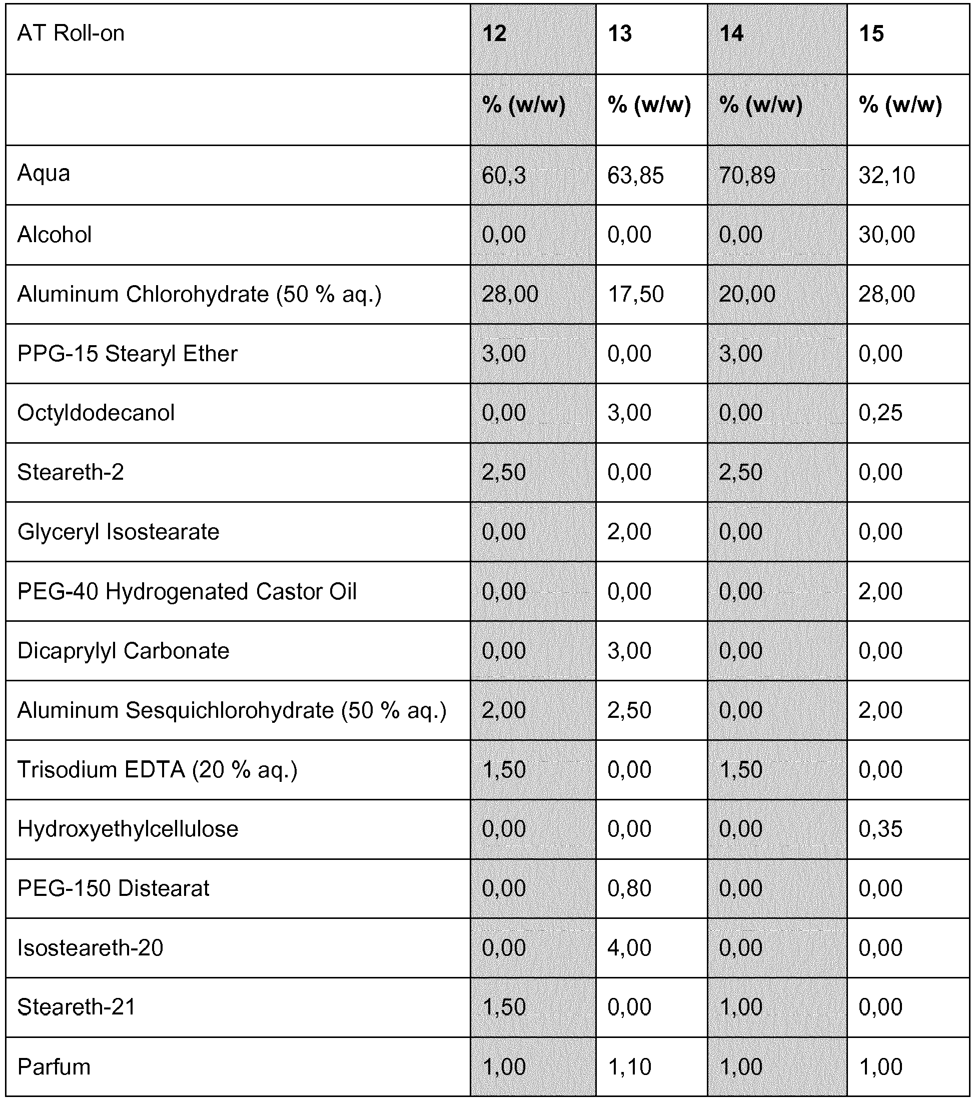

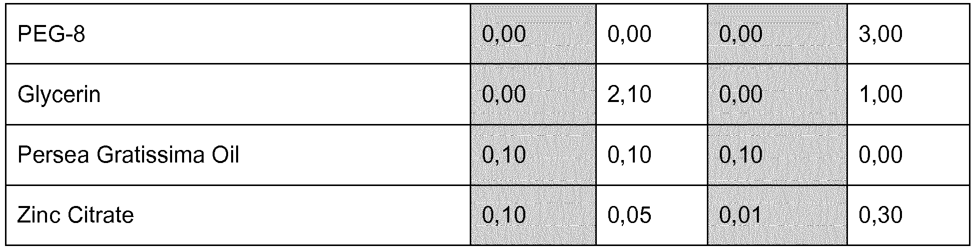

## Patentansprüche

1. Verwendung von Aluminiumsalzen in Kombination mit Adjuvantien der Gruppe
b. der Zinksalze und
c. weiterer Aluminium-basierender Antitranspirantien
in kosmetischen oder dermatologischen Zubereitungen zur Verminderung oder Verhinderung des Stress-Schwitzens entsprechend dem TSST-Design,
**dadurch gekennzeichnet, dass** als Aluminiumsalz Aluminiumchlorohydrat (ACH) und als Adjuvans der Gruppe c. Aluminium Sesquichlorohydrate gewählt werden.

2. Verwendung nach Anspruch 1 **dadurch gekennzeichnet, dass** als Adjuvantien b.) ein oder mehrere Zinksalze der Essigsäure, Milchsäure und/oder der Gruppe der Fruchtsäuren gewählt werden.

3. Verwendung nach Anspruch 2 **dadurch gekennzeichnet, dass** als Zinksalz b.) Zinkcitrat gewählt wird.

4. Verwendung nach einem der vorstehenden Ansprüche **dadurch gekennzeichnet, dass** als Aluminiumsalz Aluminiumchlorohydrat (ACH), als Adjuvans der Gruppe c. Aluminium Sesquichlorohydrate und als Adjuvans der Gruppe b. Zinkcitrat gewählt werden.

## Claims

1. Use of aluminium salts in combination with adjuvants of the group
b) of zinc salts and
c) further aluminium-based antiperspirants in cosmetic or dermatological preparations for reducing or preventing stress perspiration in accordance with the TSST design,
**characterized in that** aluminium chlorohydrate (ACH) is selected as aluminium salt and aluminium sesquichlorohydrate is selected as adjuvant of group c.

2. Use according to Claim 1, **characterized in that** one or more zinc salts of acetic acid, lactic acid and/or the group of fruit acids are selected as adjuvants b.).

3. Use according to Claim 2, **characterized in that** zinc citrate is selected as zinc salt b.).

4. Use according to any of the preceding claims, **characterized in that** aluminium chlorohydrate (ACH) is selected as aluminium salt, aluminium sesquichlorohydrate is selected as adjuvant of group c. and zinc citrate is selected as adjuvant of group b.

## Revendications

1. Utilisation de sels d'aluminium en combinaison avec des adjuvants du groupe constitué par :
b. les sels de zinc et
c. d'autres anti-transpirants à base d'aluminium, dans des préparations cosmétiques ou dermatologiques pour réduire ou inhiber la transpiration liée au stress conformément au TSST,
**caractérisée en ce que** le chlorohydrate d'aluminium (ACH) est choisi en tant que sel d'aluminium et le sesquichlorohydrate d'aluminium en tant qu'adjuvant du groupe c.

2. Utilisation selon la revendication 1, **caractérisée en ce qu'**un ou plusieurs sels de zinc de l'acide acétique, de l'acide lactique et/ou du groupe des acides de fruit sont choisis en tant qu'adjuvants b.).

3. Utilisation selon la revendication 2, **caractérisée en ce que** le citrate de zinc est choisi en tant que sel de zinc b.).

4. Utilisation selon l'une quelconque des revendications précédentes, **caractérisée en ce que** le chlorohydrate d'aluminium (ACH) est choisi en tant que sel d'aluminium, le sesquichlorohydrate d'aluminium en tant qu'adjuvant du groupe c. et le citrate de zinc en tant qu'adjuvant du groupe b.
